(19) Europäisches Patentamt
European Patent Office
Office européen des brevets

(11) **EP 1 270 679 B2**

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**09.06.2021   Patentblatt 2021/23**

(45) Hinweis auf die Patenterteilung:
**05.12.2007   Patentblatt 2007/49**

(21) Anmeldenummer: **02006873.0**

(22) Anmeldetag: **26.03.2002**

(51) Int Cl.:
*C09B 67/00* (2006.01)          *A23L 1/27* (2006.01)
*A23L 2/58* (2006.01)           *D06P 3/54* (2006.01)
*A61K 31/525* (2006.01)         *C09D 11/00* (2014.01)
*A23L 1/303* (2006.01)          *C12P 25/00* (2006.01)
*A23L 1/275* (2006.01)

(54) **Farbstoff-Gemisch zur Verwendung in Lebensmitteln, Pharmazeutika und Kosmetika**

Dye composition for use in food, pharmaceuticals and cosmetics

Composition colorante pour son utilisation dans les aliments, les produits pharmaceutiques et cosmétiques

(84) Benannte Vertragsstaaten:
**AT BE CH CY DE DK ES FI FR GB GR IE IT LI LU MC NL PT SE TR**

(30) Priorität: **01.06.2001   DE 20109224 U**

(43) Veröffentlichungstag der Anmeldung:
**02.01.2003   Patentblatt 2003/01**

(73) Patentinhaber: **Sensient Colors Europe GmbH 21502 Geesthacht (DE)**

(72) Erfinder: **Klingenberg, Andreas, Dr. 21465 Reinbek (DE)**

(74) Vertreter: **RGTH Patentanwälte PartGmbB Neuer Wall 10 20354 Hamburg (DE)**

(56) Entgegenhaltungen:
WO-A-00/70967          WO-A-97/26802
US-A- 3 206 316        US-A- 4 307 117
US-A- 4 522 743        US-A- 4 726 955
US-A- 5 139 800        US-A- 5 827 539
US-A- 5 993 880        US-A- 6 007 856
US-A- 6 132 790

Bemerkungen:
Die Akte enthält technische Angaben, die nach dem Eingang der Anmeldung eingereicht wurden und die nicht in dieser Patentschrift enthalten sind.

**Beschreibung**

Die Erfindung betrifft ein Verfahren zur Herstellung eines Farbstoff-Gemisches, das so hergestellte Farbstoff-Gemisch sowie dessen Verwendung in Lebensmitteln, Pharmazeutika und Kosmetika.

Gemische von Farbstoffen, insbesondere in flüssiger Form, werden in vielen Bereichen zur farblichen Gestaltung von Produkten verwendet. Auch bei Lebensmitteln, Pharmazeutika und Kosmetika sind flüssige Farbstoff-Gemische üblich. Hierbei sind allerdings besondere gesetzliche Vorschriften zu beachten, wie nationale Vorschriften oder Vorschriften der Europäischen Union. Besonders bei Lebensmitteln, Pharmazeutika und Kosmetika gibt es vielfältige Bestimmungen; die Verwendung von und Farbstoffen bzw. Farbstoff Gemischen bedarf vor ihrer Markteinführung der Zulassung durch die entsprechenden Behörden.

Bei den unterschiedlichen Einsatzgebieten derartiger Gemische sind je nach den Produkterfordernissen und im Hinblick auf die Eigenschaften des Endproduktes spezifische Eigenschaften der Gemische im Zusammenwirken mit anderen Komponenten zu berücksichtigen. Eigenschaften, insbesondere bei Lebensmitteln, wie Freisetzungsgeschwindigkeit, Migrationsverhalten oder Lichtstabilität bestimmen in entscheidendem Maße die Produktqualität.

Besonders im Gesundheitswesen und im Lebensmittelsektor herrscht ein starker Konkurrenz und damit Kostendruck. Außerdem fordert der Verbraucher heute in erhöhtem Maße Produkte, die visuell ansprechend sind und dem Anspruch an einen hohen Qualitätsstandard gerecht werden.

Die US-A-3 206 316 offenbart ein Verfahren zur Herstellung von in Wasser dispergierbaren Carotinoid-Präparaten, das folgende Schritte umfasst: Eine Lösung von Carotinoid-Färbemittel wird in einem organischen Carotinoid-Lösungsmittel, mit einem Salz eines höheren Fettsäureesters von Ascorbinsäuren bereitet. Aus dem erhaltenen Gemisch wird das Carotinoid-Lösungsmittel entfernt, wobei die höhere Fettsäure über 12 bis 20 Kohlenstoffatome verfügt, wobei das genannte Salz aus der Gruppe ausgewählt wurde, die aus Alkalimetall- und Aminosäuresalzen besteht. Die dabei eingesetzte höhere Fettsäure enthält 12 bis 20 Kohlenstoffatome. Die nach diesem Verfahren erhaltenen wasserdispergierbaren, carotinoid-haltigen Verbindungen enthalten langkettige Ascorbin-Fettsäureester als oberflächenaktive Substanzen. Des weiteren enthalten die Verbindungen Kolloide.

Einen Prozess zur Herstellung einer Öl/Wasser-Dispersion mit beta-Carotin als Farbstoff, die für Getränke verwendet werden kann, beschreibt die US-A-6,007,856. Diese Öl-in-Wasser-Dispersionen von beta-Carotin oder anderen Carotinoiden, die oxidationsbeständig sind, werden in Anwesenheit von Polyphosphaten, insbesondere von Natriumhexametaphosphat, durch Kontaktieren eines in Wasser dispergierbaren Beadlet, das mindestens ungefähr 5 % kolloidales Carotinoid enthält, mit der wässrigen Phase bereitet, um Tröpfchen des Carotinoids auszubilden, die in engem Kontakt mit einer genügenden Öl-Phase stehen, damit das Carotinoid oxidationsbeständig ist, und zwar sogar in Abwesenheit eines wirksamen Antioxidans, wie etwa Ascorbinsäure. Diese Öl-in-Wasser-Carotinoid-Dispersionen werden in verdünnten Saftgetränken verwendet, um Vitamin-A-Gehalt und Farbe zu verstärken, wie auch in anderen Öl/Wasser-Lebensmittel- und Getränkeprodukten, in denen Verstärkung von Vitamin-A-Gehalt und Farbe erwünscht sind.

Fein verteilte, pulverförmige Carotinoid- oder Retinoid-Gemische, in denen das Carotinoid oder Retinoid eine Teilchengröße von weniger als 0,5 Mikron hat, werden nach US-A-4,522,743 durch ein Verfahren bereitet, in dem ein Carotinoid oder Retinoid in einem flüchtigen, mit Wasser mischbaren, organischen Lösungsmittel bei zwischen 50 °C und 200 °C, wenn nötig unter Überdruck, in weniger als 10 Sekunden gelöst wird. Das Carotinoid wird sofort in einer kolloidal dispergierten Form aus der molekular verteilten Lösung durch rasches Mischen der letztgenannten mit einer wässrigen Lösung eines quellfähigen Kolloids bei zwischen 0° C und 50° C ausgefällt und die erhaltene Dispersion in herkömmlicher Weise vom Lösungsmittel und vom Dispergiermittel befreit.

Nach der US-A-5,827,539 ist ein Verfahren zur Herstellung eines trockenen Pulvers eines Carotinoides bekannt, das folgende Schritte umfaßt: Dispergieren eines Carotinoids in einem Öl zur Herstellung einer Carotinoid-Öl-Dispersion; Mahlen der Carotinoid-Öl-Dispersion zur Herstellung einer gemahlenen Carotinoid-Öl-Dispersion; Lösen eines Stärke-Verkapselungsmittels, eines Zuckers und eines Antioxidans in Wasser zur Herstellung eines Verkapselungsgemisches; Mischen der gemahlenen Carotinoid-Öl-Dispersion und des Verkapselungsgemisches zur Bereitung einer Emulsion, und Trocknen der Emulsion zur Herstellung eines Pulvers, wodurch das Verkapselungsgemisch die gemahlene Carotinoid-Öl-Dispersion einschließt. Danach wird dieses trockene Pulver, das Carotinoi-de enthält, die durch Mahlen eines Gemisches aus Carotinoiden und Öl zur Verringerung der Carotinoid-Teilchengröße durch Emulgieren des Gemisches mit einem Verkapselungsgemisch und Trocknen der Emulsion er-zeugt. Das Verkapselungsgemisch enthält daher ein Stärke-Verkapselungsmittel, einen Zucker und ein Antioxidans. Das erhaltene, in Wasser dispergierbare Pulver enthält einen hohen Anteil an Carotinoiden, ist dabei aber vor Oxidation geschützt.

Gewichtsverhältnis von Kupferchlorophyllin zum hydrophilen oberflächenaktiven Mittel liegt dabei im Bereich von ungefähr 1:1 bis 1:6. Für Getränke- und Gelatinedessertmischungen werden Geschmacksstoffe und ausreichend Säure verwendet, um einen pH-Wert von weniger als 4 zu erhalten, wenn das trockene Gemisch mit Wasser gemischt wird.

Fein verteilte, pulverförmige Carotinoid- oder Retinoid-Gemische, in denen das Carotinoid oder Retinoid eine Teilchengröße von weniger als 0,5 Mikron hat, werden nach US-A-4,522,743 durch ein Verfahren bereitet, in dem ein Carotinoid oder Retinoid in einem flüchtigen, mit Wasser mischbaren, organischen Lösungsmittel bei zwischen 50° C und 200° C, wenn nötig unter Überdruck, in weniger als 10 Sekunden gelöst wird. Das Carotinoid wird sofort in einer kolloidal dispergierten Form aus der molekular verteilten Lösung durch rasches Mischen der letztgenannten mit einer wässrigen Lösung eines quellfähigen Kolloids bei zwischen 0° C und 50° C ausgefällt und die erhaltene Dispersion in herkömmlicher Weise vom Lösungsmittel und vom Dispergiermittel befreit.

Gegenstand der US-A-4,726,955 ist ein Carotinoid- oder Retinoid-Gemisch entsprechend der US-A-4,522,743, jedoch wird bei der Herstellung des Gemisches als Kolloid Milch oder Magermilch oder eine wässrige Lösung von Trockenmilch verwendet.

Die WO 0070967 A offenbart ein Gemisch, das Farbstoffteilchen umfasst, die mindestens teilweise mit Rübenpektin, Zichorienpektin und/oder Topinamburpektin oder anderen Pektinarten mit hohem Acetylierungsgrad beschichtet sind. Das Gemisch, das in Wasser dispergierbar ist, soll in der Herstellung von Gesundheitsförderungsmitteln und/oder Färbemitteln zum Gebrauch beim Färben essbarer Produkte einschließlich Lebensmitteln und Nutraceuticals verwendbar und zum Färben von pharmazeutischen Produkten geeignet sein.

Nach der US-A-5,827,539 ist ein Verfahren zur Herstellung eines trockenen Pulvers eines Carotinoides bekannt, das folgende Schritte umfasst: Dispergieren eines Carotinoids in einem Öl zur Herstellung einer Carotinoid-Öl-Dispersion; Mahlen der Carotinoid-Öl-Dispersion zur Herstellung einer gemahlenen Carotinoid-Öl-Dispersion; Lösen eines Stärke-Verkapselungsmittels, eines Zuckers und eines Antioxidans in Wasser zur Herstellung eines Verkapselungsgemisches; Mischen der gemahlenen Carotinoid-Öl-Dispersion und des Verkapselungsgemisches zur Bereitung einer Emulsion, und Trocknen der Emulsion zur Herstellung eines Pulvers, wodurch das Verkapselungsgemisch die gemahlene Carotinoid-Öl-Dispersion einschließt. Danach wird dieses trockene Pulver, das Carotinoide enthält, die durch Mahlen eines Gemisches aus Carotinoiden und Öl zur Verringerung der Carotinoid-Teilchengröße durch Emulgieren des Gemisches mit einem Verkapselungsgemisch und Trocknen der Emulsion erzeugt. Das Verkapselungsgemisch enthält daher ein Stärke-Verkapselungsmittel, einen Zucker und ein Antioxidans. Das erhaltene, in Wasser dispergierbare Pulver enthält einen hohen Anteil an Carotinoiden, ist dabei aber vor Oxidation geschützt.

Die US-A-4,307,117 beschreibt die Stabilisierung von Kurkumin gegen Farbveränderungen, indem das Kurkumin selbst bei niedrigen stabilen pH-Werten gehalten wird, ohne dass dabei das pH-Gleichgewicht eines trockenen Nahrungsmittel-Gemisches umzukippen vermag, das mit ihm in Berührung gebracht werden könnte. Der pH-Wert des Kurkumins wird auf einem Wert im Bereich von zwischen ungefähr 3,5 und ungefähr 4,5 gehalten, und umfasst ein sprühgetrocknetes, inniges Gemisch aus Kurkumin, einer organischen Säure, einem Puffer, einem Dispergiermittel für das Kurkumin und einem filmbildenden Verkapselungsmittel. Der Farbstoff soll bevorzugt in trockenen Gemischen für Fertigpuddings, Verwendung finden, die aufgrund der Salze, die verwendet werden, um das Erstarren zu bewirken, alkalisch sind.

Gegenstand der US-A-6 132 790 ist ein stabiles Carotinoid-Gemisch, das ein Carotinoid in einem ölartigen Lösungsmittel, wobei das Gewicht des Carotinoids im Carotinoidgemisch bis zu 12 % beträgt, eine Dispersion einer in Wasser dispergierbaren Matrix und eines Stabilisators und optional eines nicht ölartigen Lösungsmittels, und einen Emulgator umfasst, wobei Carotinoid, nicht ölartiges Lösungsmittel, in Wasser dispergierbare Matrix, Stabilisator und Emulgator alle in einer Form liegen, in der sie in der Natur vorkommen oder aus natürlichen Quellen ohne Verwendung synthetischer Verbindungen abgeleitet worden sind.

Der Erfindung liegt deshalb die Aufgabe zugrunde ein Verfahren zur Herstellung eines Farbstoff-Gemisches, das leicht herstellbar; qualitativ hochwertig und leicht zu verarbeiten ist, zu vorteilhaften Produkteigenschaften führt und bei dessen Herstellung keine organischen Lösungsmittel verwendet werden, bereitzustellen. Insbesondere ist es eine Aufgabe Farbstoff-Gemische für Lebensmittel mit vorteilhaften Eigenschaften hinsichtlich Freisetzungsgeschwindigkeit, Ergiebigkeit, Migrationsverhalten, Auflösungsgeschwindigkeit und Lichtstabilität bereitzustellen.

Diese Aufgabe wird durch die in Anspruch 1 genannten Merkmale gelöst.

Nach Maßgabe der Erfindung ist Verfahren zur Herstellung eines Farbstoff-Gemisches zur Verwendung in Lebensmitteln, Pharmazeutika und Kosmetika vorgesehen, wobei das Gemisch

a.) eine Farbstoff-Dispersion mit einem Feststoff, der ein Carotinoid wie Carotine und Carotinoide sowohl naturidentischen wie auch natürlichen Ursprungs, ein Betanin, ein Riboflavin, ein Anthocyan, ein Carminprodukt, ein Curcuminoid, ein Porphyren und/oder eine Chlorophyllverbindung, eine Chlorophillinverbindung, eine Kupfer-Chlorophyll- und/oder Kupfer-Chlorophillinverbindung ist, mit einer durchschnittlichen Teilchengröße von weniger als 30 $\mu$m und mit einem Hilfs- oder Zusatzstoff, der ein Zucker, ein Polysaccharid, ein Hydrokolloid und Wasser oder ein Antioxidant oder Konservierungsmittel ist, und

b.) eine oberflächenaktive Substanz, die ein in Lebensmitteln bzw. Lebensmittelzusatzstoffen, wie Farbstoffe, zugelassener Emulgator nämlich ein Lecithin, Polysorbat 80, Lactem oder Citrem ist,

umfasst, wobei die oberflächenaktive Substanz in dem Farbstoff-Gemisch in einem Anteil von unter 20 %, vorzugsweise von unter 10 % und am meisten bevorzugt von 1 % bis 5 % bezogen auf das Gemisch enthalten ist, wobei das Verfahren dadurch gekennzeichnet ist, dass

a.) in einer ersten Stufe die Farbstoff-Dispersion durch Mischen bei einer Temperatur von 30 °C bis 60 °C mindestens eines Farbstoffes, mit mindestens einem Lösungsmittel und gegebenenfalls mit weiteren Bestandteilen zu einer Dispersion und anschließender Zerkleinerung der Mischung durch apparative Dispergierer und Vermahlinstrumente, wie Mühlen, Turrax-Homogenisatoren oder andere leistungsfähige Rührinstrumente, zu einer durchschnittlichen Teilchengröße von weniger als 30 $\mu$m hergestellt wird, danach

b.) in einer zweiten Stufe die oberflächenaktive Substanz bei einer Temperatur von 40 °C bis 80 °C z. B. durch eine wässrige Auflösung bereitgestellt wird, wonach

c.) in einer dritten Stufe die Zugabe der in der zweiten Stufe hergestellten oberflächenaktiven Substanz zu der in der ersten Stufe hergestellten auf 30 °C bis 60 °C temperierten Farbstoff-Dispersion bei einer Temperatur von 30 °C bis 60 °C erfolgt, wobei zur Herstellung des Farbstoff-Gemisches keine organischen Lösungsmittel verwendet werden.

Ferner ist ein Farbstoff-Gemisch, das mittels des erfindungsgemäßen Verfahrens hergestellt wurde, vorgesehen.

Danach besteht die Erfindung in einem in Lebensmitteln, pharmazeutischen und Kosmetika verwendbaren, qualitativ hochwertigen und leicht zu verarbeitenden Farbstoff-Gemisch aus einer Farbstoff-Dispersion und einer oberflächenaktiven Substanz, wobei die Dispersion den Farbstoff in Feststoff enthält, der beispielsweise ein Carotinoid wie Carotine und Carotinoide sowohl naturidentischen wie auch natürlichen Ursprungs, ein Betanin, ein Riboflavin, ein Anthocyan, ein Carminprodukt, ein Curcuminoid, ein Porphyren und/oder eine Chlorophyllverbindung, eine Chlorophillinverbindung, eine Kupfer-Chlorophyll- und/oder Kupfer-Chlorophillinverbindung ist.

Die Eigenschaften des erfindungsgemäßen Gemisches konnten durch eine Auswahl und die Kombination von geeigneter Farbstoff-Dispersion und vorteilhafter oberflächenaktiver Substanz in Bezug auf Freisetzungsgeschwindigkeit, Ergiebigkeit, Migrationsverhalten, Auflösungsgeschwindigkeit und Lichtstabilität überraschender Weise in hohem Maß optimiert und signifikant verbessert werden.

Insbesondere konnte in dem erfindungsgemäßen Gemisch die Ergiebigkeit in dem jeweiligen Medium der Endanwendung, wie gefärbten flüssigen Lebensmitteln, z. B. Getränkezubereitungen, gegenüber bekannten Farbstoff-Gemischen verbessert werden. Eine verbesserte Ergiebigkeit ist deshalb von Bedeutung, weil die Ergiebigkeit ein entscheidender Qualitätsparameter ist und ein verbessertes Freisetzungsverhalten einen geringeren Produkteinsatz und damit ökonomischere Rezepturen erlaubt. Dies führt nicht zuletzt zu einer erheblichen Kostenreduzierung.

Überraschender Weise konnte mit dem erfindungsgemäßen Gemisch auch ein unerwünschtes "Ausbluten" und der Übertritt von Farbstoffen zwischen unterschiedlichen Phasen, z. B. Fett und Zucker, in Lebensmitteln gezielt vermindert oder sogar ganz vermieden werden. Vorteilhaft an dem erfindungsgemäßen Gemisch ist, dass es mit ihm möglich wird, einen bestimmten Farbstoff oder eine Farbstoffkombination im wesentlichen ausschließlich in einer gewünschten Phase zu verteilen und zu verhindern, dass es zu einem erkennbaren Phasenübertritt des oder der Farbstoffe kommt.

Außerdem zeigt das erfindungsgemäße Gemisch eine rasche Auflösung in dem Medium der Endanwendung. Dies hat den Vorteil, dass die Herstellungszeiten verkürzt und so die Produktionsabläufe optimiert und andere Weiterverarbeitungsschritte schneller und effizienter eingestellt werden können. Dies führt schließlich auch zu einer Einsparung von Maschinenzeiten und Personaleinsatzzeiten, wodurch eine Kostenreduzierung möglich wird.

Schließlich ist die Lichtstabilität bei bekannten Farbstoff-Gemischen ein kritischer Punkt. Insbesondere bei natürlichen Farbstoff-Gemischen ist die Lichtstabilität außerordentlich begrenzt und erfüllt häufig nicht die Anforderungen für einen Einsatz, besonders nicht in Lebensmitteln wie Getränken. Dieser Sachverhalt macht den Einsatz von weiteren Stabilisationsmaßnahmen erforderlich, durch die z. B. ungesättigte Strukturen gegen Oxidation geschützt werden. Hier zeigen die erfindungsgemäßen Gemische hervorragende Verbesserungen und erlauben eine Verminderung derartiger Stabilisatoren oder machen den Einsatz von ihnen gänzlich überflüssig. Andererseits können nun Farbstoffe verwendet werden, die früher nicht eingesetzt werden konnten.

Als besonders vorteilhaft hat es sich erwiesen, wenn das erfindungsgemäße Gemisch eine mittlere Teilchengröße von kleiner 25 $\mu$m, besonders bevorzugt von kleiner 20 $\mu$m und am meisten bevorzugt von kleiner 10 $\mu$m hat.

Der Farbstoff liegt in dem Gemisch geeigneter Weise als Feststoff vor. Der Farbstoff kann ein Farbstoff auf Naturbasis sein, ein modifizierter Farbstoff auf Naturbasis oder ein synthetischer Farbstoff. Besonders bevorzugt sind Farbstoffe auf Naturbasis, wie Carotinoid, Betanin, Riboflavin, Anthocyan, Carminprodukte, Curcuminoid oder Chlorophyllverbindungen, Chlorophillinverbindungen sowie Kupfer-Chlorophyll- und/oder Kupfer-Chlorophillinverbindungen, wobei unter Carotinoide, Carotine und Carotinoide, sowohl naturidentischen wie auch natürlichen Ursprungs fallen.

Weiterhin kann das Gemisch weitere Hilfs- oder/und Zusatzstoffe enthalten, die in der Lebensmittelindustrie, der pharmazeutischen Industrie oder der Kosmetikindustrie üblich sind, wie Geschmacksverstärker, Duftstoffe oder Konservierungsstoffe sowie Trägerstoffe, wie Polysaccharide, Maltodextrine und Polyole, und die Eigenschaften des erfindungsgemäßen Gemisches nicht negativ beeinflussen oder zu einer Verbesserung der Eigenschaften führen.

Besonders vorteilhaft ist, wenn die oberflächenaktive Substanz in dem erfindungsgemäßen Gemisch in einem Anteil von unter 20 %, vorzugsweise von unter 10 % und am meisten bevorzugt von 1 bis 5 % bezogen auf das Gemisch enthalten ist, wobei sich die % auf Gew.-% beziehen.

Neben den genannten Komponenten können weitere geeignete Bestandteile in dem Gemisch enthalten sein, die zu einer weiteren Verbesserung oder einem vorteilhaften Erscheinungsbild des Endproduktes beitragen.

Bei dem Verfahren zur Herstellung des Farbstoff-Gemisches wird in einem ersten Schritt eine erste Komponente, nämlich eine Farbstoff-Dispersion, und in einem zweiten Schritt eine zweite Komponente, nämlich eine oberflächenaktive Substanz, hergestellt. Diese beiden Komponenten werden anschließend vorzugsweise durch intensives Verrühren homogen vermischt. Das Mischen wird vorzugsweise bei erhöhter Temperatur, insbesondere bei 30° bis 60° C durchgeführt.

Durch die Verfahrensbedingungen des erfindungsgemäßen Verfahrens wird vorteilhafter Weise ein Gemisch erhalten, das bei seiner Verwendung zu vorteilhaften Eigenschaften hinsichtlich Freisetzungsgeschwindigkeit, Migrationsverhalten oder Lichtstabilität führt. Hinzukommt, dass bei der Durchführung des Verfahrens kein organisches Lösungsmittel eingesetzt wird. Auch keine Öle oder Fette werden verwendet. Bevorzugterweise wird von organischen bzw. natürlichen Pigmenten ausgegangen.

Insbesondere die Abfolge der einzelnen Verfahrensschritte und die gewählten Temperaturbereiche und die Zeitdauer der einzelnen Verfahrensschritte haben sich als vorteilhaft für die Herstellung des erfindungsgemäßen Gemisches und seiner Eigenschaften erwiesen.

Besonders bevorzugt ist eine Mischtemperatur beim Vermischen von Farbstoff-Dispersion und oberflächenaktiver Substanz von 30° bis 50° C, vorzugsweise bei 40° bis 50° C und eine Mischzeit von 15 bis 90 Minuten, besonders bevorzugt von 30 bis 60 Minuten.

Vorteilhaft zeigte sich auch, wenn die Farbstoff-Dispersion bei Zugabe der zweiten Komponente temperiert wurde auf 30° bis 50° C, vorzugsweise auf 35° bis 45° C und am meisten bevorzugt auf ca. 40° C.

Es ist möglich das erfindungsgemäße Gemisch in allen bekannten Lebensmitteln, Pharmazeutika oder Kosmetika einzusetzen. Die vorteilhaften Eigenschaften werden insbesondere in Lebensmittel mit flüssiger und auch mit fester Konsistenz erreicht.

Die Erfindung wird durch die folgenden Beispiele veranschaulicht.

**Beispiel 1**

Herstellung eines erfindungsgemäßen Farbstoff-Gemisches

1.1. Herstellung der Farbstoff-Dispersion (Komponente A)

Ca. 300 g Gummi arabicum werden unter Rühren in einer Lösung ca. 400 g demineralisiertem Wasser und ca. 100 g Maltodextrin bei 40° bis 50°C für 30 bis 60 Minuten gelöst, bis eine homogene Mischung vorliegt.

Anschließend werden dieser Mischung ca. 100 g Curcumin-Pulver zugesetzt und verrührt. Dieses Gemisch wird in einer Dispersionsmühle vermahlen bis die durchschnittliche Teilchengröße in dieser Suspension ca. 10 μm erreicht hat.

1.2. Herstellung der oberflächenaktiven Substanz und des Endproduktes (Komponente B)

Die Komponente A wird unter Rühren auf ca. 40° C temperiert und als Emulgator eine bei einer Temperatur von 60° bis 80° C hergestellte Lösung aus ca. 100 g Wasser und ca. 10 g Citrem, Zitronensäureester von Monoglyceriden (E-472c) zugesetzt. Die Mischung wird weiterhin für ca. 30 Minuten bei ca. 40° bis 50° C verrührt Die erhaltene Suspension stellt das erfindungsgemäße Gemisch dar.

**Beispiel 2**

Die gemäß Beispiel 1 hergestellten Komponenten A sowie das erfindungsgemäße Gemisch, bestehend aus Komponente A und B, wurden hinsichtlich ihrer Eigenschaften nun weiter untersucht.

2.1. Das erfindungsgemäße Produkt hat ein vorteilhaftes Freisetzungsverhalten und gute Ergiebigkeit

Um das Freisetzungsverhalten und die Ergiebigkeit von Produkten mit gleicher Teilchengrößenverteilung bewerten und quantifizieren zu können, wurde folgende Methode zur Berechnung der Freisetzung entwickelt:

20 bis 50 g des zu untersuchenden Curcumin-Produktes werden in 100 ml einer 50 %igen Zuckerlösung homogen verteilt und die Farbintensität wird als Lab - Wert dieser Lösung bestimmt. Aus den Zahlenwerten der Konzentration, des spezifischen Absorptionswertes E1 % (Farbwert), des L - und des b - Wertes wird die Freisetzung nach folgender Gleichung berechnet:

$$\text{Freisetzung} = \frac{b - \text{Wert}\,.10\,[g/ml] \times \text{Volumen}\,[ml]}{L - \text{Wert} \times \text{Einwaage}\,[g] \times \text{Farbwert}}$$

Die Untersuchung der beiden Produkte Komponente A und erfindungsgemäßes Gemisch aus Komponente A und B erbrachte folgendes Ergebnis:

|  |  |
|---|---|
| Freisetzung der Komponente A: | 20 |
| Freisetzung erfindungsgemäßes Gemisch: | 30 |

Somit zeigt das erfindungsgemäße Gemisch eine um 50 % verbesserte Freisetzung gegenüber dem Produkt (Komponente A). Die erfindungsgemäße Kombination von Farbstoff-Dispersion mit einer oberflächenaktiven Substanz ist also bekannten Farbstoff-Dispersionen hinsichtlich ihres Freisetzungsverhaltens überlegen.

2.2. Das erfindungsgemäße Produkt hat ein vorteilhaftes Migrationsverhalten

Die gemäß Beispiel 1 hergestellten Produkte wurden hinsichtlich des Migrationsverhaltens in folgendem Testmodell untersucht:

Mit Curcumin gefärbte weiße Schokolade (0,1 g des erfindungsgemäßen Gemisches bzw. von Komponente A alleine gemäß Beispiel 1 auf 5 g weiße Schokolade wurden verglichen) wurde wie unter 2.2. beschrieben auf befeuchtetes Filterpapier getropft. Auch hier kam es zu einer unterschiedlich starken Verschleppung des Farbstoffes in das Filterpapier durch die mobile Phase Wasser. Komponente A bewirkte eine deutliche bis intensive Färbung des Filterpapiers, wohingegen das erfindungsgemäße Gemisch das Filterpapier nur geringfügig färbte.

Das erfindungsgemäße Gemisch zeichnet sich also durch deutlich verbesserte Migrationseigenschaften aus.

2.3. Das erfindungsgemäße Produkt hat eine vorteilhafte Auflösungsgeschwindigkeit

Die Auflösungsgeschwindigkeit von Komponente A und des erfindungsgemäßen Gemisches gemäß Beispiel 1 wurden in einer wässrigen Lösung untersucht.

Hierzu wurde 0,1 g Curcumin-Produkt gemäß Beispiel 1 in 100 ml demineralisiertem Wasser unter Rühren bei 300 Upm mit einem 5 cm Rührstab bei 20° C in ein Becherglas (250 ml) gegeben. Die Zeit bis zum vollständigen Auflösen (kein Bodensatz erkennbar) von Komponente A bzw. des erfindungsgemäßen Gemisches wurde gemessen. Komponente A benötigte 280 bis 290 Sekunden. Dagegen benötigte das erfindungsgemäße Gemisch lediglich 230 bis 240 Sekunden.

Somit zeigte das erfindungsgemäße Produkt im Vergleich zu Komponente A ein um ca. 20 % verbessertes Auflösungsverhalten.

2.4. Das erfindungsgemäße Produkt hat eine vorteilhafte Lichtstabilität

Zur Bestimmung der Lichtstabilität wurde Komponente A und das erfindungsgemäße Gemisch gemäß Beispiel 1 in wässriger Auflösung in einer Konzentration von 20 bis 40 mg / 250 ml mit einem "Suntest" - Gerät einer Bestrahlung unterzogen. Der Farbabbau wurde mittels einer Farbtonmessung im oben beschriebenen L a b -System während der gesamten Bestrahlungsdauer protokolliert. Als bestimmende Messgröße wurde der Zeitpunkt bis zu einem Verlust von 30 % der Farbintensität (b - Wert) der wässrigen Auflösung festgelegt.

Bei den untersuchten Proben wurden folgende Messwerte festgestellt:

| Komponente A: | 30 % Farbabbau nach 150 Minuten |
|---|---|
| Erfindungsgemäßes Gemisch: | 30 % Farbabbau nach 290 Minuten |

Somit zeigte das erfindungsgemäße Gemisch im Vergleich zu Komponente A eine um nahe zu 100 % verbesserte Lichtstabilität.

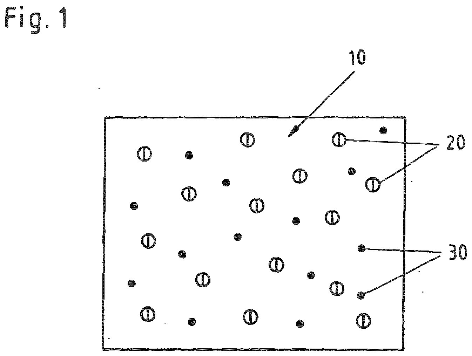

Das erfindungsgemäße Gemisch ist in Fig. 1 in einer vergrößerten Wiedergabe dargestellt, wobei das aus einer Farbstoff-Dispersion 20 und einer oberflächenaktiven Substanz 30 bestehende Gemisch mit 10 bezeichnet ist.

## Patentansprüche

1. Verfahren zur Herstellung eines Farbstoff-Gemisches zur Verwendung in Lebensmitteln, Pharmazeutika und Kosmetika, wobei das Gemisch

   a.) eine Farbstoff-Dispersion mit einem Feststoff, der ein Carotinoid wie Carotine und Carotinoide sowohl naturidentischen wie auch natürlichen Ursprungs, ein Betanin, ein Riboflavin, ein Anthocyan, ein Carminprodukt, ein Curcuminoid, ein Porphyren und/oder eine Chlorophyllverbindung, eine Chlorophillinverbindung, eine Kupfer-Chlorophyll- und/oder Kupfer-Chlorophillinverbindung ist, mit einer durchschnittlichen Teilchengröße von weniger als 30 $\mu$m und mit einem Hilfs- oder Zusatzstoff, der ein Zucker, ein Polysaccharid, ein Hydrokolloid und Wasser oder ein Antioxidant oder Konservierungsmittel ist, und
   b.) eine oberflächenaktive Substanz, die ein in Lebensmitteln bzw. Lebensmittelzusatzstoffen, wie Farbstoffen, zugelassener Emulgator nämlich ein Lecithin, Polysorbat 80, Lactem oder Citrem ist,

   umfasst, wobei die oberflächenaktive Substanz in dem Farbstoff-Gemisch in einem Anteil von unter 20 %, vorzugsweise von unter 10 % und am meisten bevorzugt von 1 % bis 5 % bezogen auf das Gemisch enthalten ist, wobei das Verfahren **dadurch gekennzeichnet** ist,

   a.) in einer ersten Stufe die Farbstoff-Dispersion durch Mischen bei einer Temperatur von 30 °C bis 60 °C mindestens eines Farbstoffes, mit mindestens einem Lösungsmittel und gegebenenfalls mit weiteren Bestandteilen zu einer Dispersion und anschließender Zerkleinerung der Mischung durch apparative Dispergierer und Vermahlinstrumente, wie Mühlen, Turrax-Homogenisatoren oder andere leistungsfähige Rührinstrumente, zu einer durchschnittlichen Teilchengröße von weniger als 30 $\mu$m hergestellt wird, danach
   b.) in einer zweiten Stufe die oberflächenaktive Substanz bei einer Temperatur von 40 °C bis 80 °C z. B. durch eine wässrige Auflösung bereitgestellt wird, wonach
   c.) in einer dritten Stufe die Zugabe der in der zweiten Stufe hergestellten oberflächenaktiven Substanz zu der in der ersten Stufe hergestellten auf 30 °C bis 60 °C temperierten Farbstoff-Dispersion bei einer Temperatur von 30 °C bis 60 °C erfolgt,

   wobei zur Herstellung des Farbstoff-Gemisches keine organischen Lösungsmittel verwendet werden.

2. Verfahren nach Anspruch 1,
   **dadurch gekennzeichnet, dass**
   das Gemisch ferner Hilfs-oder/und Zusatzstoffe enthält, die aus der Gruppe ausgewählt sind, die Geschmacksverstärker, Duftstoffe oder Konservierungsstoffe umfasst.

3. Verfahren nach Anspruch 1 oder Anspruch 2,
   **dadurch gekennzeichnet, dass**
   das Gemisch ferner Trägerstoffe enthält, die aus der Gruppe ausgewählt sind, die Polysaccharide, Maltodextrine und Polyole umfasst.

4. Verfahren nach einem der vorstehenden Ansprüche,
   **dadurch gekennzeichnet,**
   **dass** die Mischtemperatur beim Vermischen von Farbstoff-Dispersion und oberflächenaktiver Substanz von 30 bis 50 °C, vorzugsweise bei 40 bis 50 °C, beträgt und die Mischzeit 15 bis 90 Minuten, vorzugsweise 30 bis 60 Minuten, beträgt.

**5.** Verfahren nach einem der vorstehenden Ansprüche,
**dadurch gekennzeichnet,**
**dass** die Farbstoff-Dispersion bei Zugabe der zweiten Komponente auf 30 bis 50 °C, vorzugsweise auf 35 bis 45 °C und am meisten bevorzugt auf 40 °C temperiert wird.

**6.** Farbstoff-Gemisch, erhalten durch das Verfahren nach einem der Ansprüche 1 bis 5, umfassend

a.) eine Farbstoff-Dispersion mit einem Feststoff, der ein Carotinoid wie Carotine und Carotinoide sowohl naturidentischen wie auch natürlichen Ursprungs, ein Betanin, ein Riboflavin, ein Anthocyan, ein Carminprodukt, ein Curcuminoid, ein Porphyren und/oder eine Chlorophyllverbindung, eine Chlorophillinverbindung, eine Kupfer-Chlorophyll- und/oder Kupfer-Chlorophillinverbindung ist, mit einer durchschnittlichen Teilchengröße von weniger als 30 $\mu$m und mit einem Hilfs- oder Zusatzstoff, der ein Zucker, ein Polysaccharid, ein Hydrokolloid und Wasser oder ein Antioxidant oder Konservierungsmittel ist, und
b.) eine oberflächenaktive Substanz, die ein in Lebensmitteln bzw. Lebensmittelzusatzstoffen, wie Farbstoffen, zugelassener Emulgator nämlich ein Lecithin, Polysorbat 80, Lactem oder Citrem ist, wobei die oberflächenaktive Substanz in dem Farbstoff-Gemisch in einem Anteil von unter 20 %, vorzugsweise von unter 10% und am meisten bevorzugt von 1 % bis 5% bezogen auf das Gemisch enthalten ist.

**Claims**

**1.** Method for production of a dye mixture for using in food, pharmaceuticals and cosmetics, whereby the mixture

a) comprises a dyestuff dispersion with a solid which is a carotenoid such as carotene and carotenoids of nature identical as well as of natural origin, a betanine, a riboflavin, an anthocyanine, a carmine product, a curcuminoid, a porphyrene and/or a chlorophyll compound, a chlorophyll in compound, a copper/chlorophyll and/or copper/chlorophyllin compound with an average particle size of less than 30 $\mu$m and with an auxiliary agent or additive which is a sugar, a polysaccharide, a hydrocolloid and water or an antioxidant agent or preservative and
b) a surface-active substance which is an emulsifying agent authorized in food or food additives such as dyestuff namely a lecithin, polysorbate 80, Lactem or Citrem.

whereby the surface-active substance is contained in the dyestuff mixture with a proportion of less than 20%, preferably of less than 10% and most preferably of 1 % to 5% with respect to the mixture, whereby the method is **characterized in that**

a) in a first stage the dyestuff dispersion is produced by mixing at a temperature of 30° to 60°C at least one dyestuff with at least one solvent and if necessary with further constituents to a dispersion and subsequent comminution of the mixture by apparative dispergators and comminution instruments such as mills, Turrax homogenizers and other powerful stirring apparatus to an average particle size of less than 30 $\mu$m, then
b) in a second stage the surface-active substance is made available at a temperature of 40°C to 80°C by an aqueous dissolution after which
c) in a third stage the addition of the surface-active substance produced in the second stage to the dyestuff dispersion produced in the first stage, tempered to 30°C to 60°C takes place, whereby no organic solvents are used for the production of the dyestuff mixture.

**2.** Method according to claim 1,
**characterized in that**
the mixture further contains auxiliary agents and/or additives which are selected from the group consisting of enhancers, aromatics or preservatives.

**3.** Method according to claim 1 or claim 2,
**characterized in that**
the mixture further contains carrier agents which are selected from the group consisting of polysaccharides, maltrodextrines and polyols.

**4.** Method according to any of the preceding claims,
**characterized in that**
the temperature of the mixture when blending the dyestuff dispersion. and the surface-active substance is of 30 to 50°C, preferably of 40 to 50°C and the mixing duration 15 to 90 minutes, preferably 30 to 60 minutes.

**5.** Method according to any of the preceding claims,
**characterized in that**
the dyestuff dispersion is tempered when adding the second component to 30-50°C, preferably to 35-45°C and most preferably to 40°C.

**6.** Dyestuff mixture obtained by the method according to any of the claims 1 to 5 comprising

a) a dyestuff dispersion with a solid which is a carotenoid such as carotene and carotenoids of nature identical as well as of natural origin, a betanine, a riboflavin, an anthocyanine, a carmine product, a curcuminoid, a porphyrene - and/or, a chlorophyll compound, a chlorophyllin compound, a copper/chlorophyll and/or copper/chlorophyllin compound with an average particle size of less than 30 μm and with an auxiliary agent or additive which is a sugar, a polysaccharide, a hydrocolloid and water or an antioxidant agent or preservative and
b) a surface-active substance which is an emulsifying agent authorized in food or food additives such as dyestuff namely a lecithin, polysorbate 80, Lactem or Citrem, whereby the surface-active substance is contained in the dyestuff mixture with a proportion of less than 20%, preferably of less than 10% and most preferably of 1% to 5% with respect to the mixture.

**Revendications**

**1.** Procédé pour la production d'un mélange colorant pour utilisation dans les aliments, les produits pharmaceutiques et cosmétiques, le mélange comprenant

a) une dispersion colorante avec un solide qui est un caroténoïde tel que du carotène et des caroténoïdes d'origine identique au nature! ou d'origine naturelle, un bétadine, un riboflavine, un anthocyane, un produit de carmine, un curcuminoïde, un porphyrène et/ou un compose de chlorophylle, un composé de chlorophylline, un composé de cuivre/chlorophylle et/ou cuivre/chlorophylline avec une grandeur de particules moyenne de mains de 30 μm et avec un agent auxiliaire ou un additif qui est un sucre, un polysaccharide, un hydrocolloïde et de l'eau ou un antioxydant ou un conservateur et
b) une surface tensioactive qui est un émulgateur autorisé dans les aliments ou les additifs alimentaires tels que les colorants, à savoir une lécithine, polysorbate 80, Lactem ou Citrem,

la substance tensioactive étant contenue dans le mélange colorant dans une proportion inférieure a 20%, de préférence inférieure a 10% et de manière particulièrement préférée de 1% à 5% par rapport au mélange.
le mélange étant, **caractérise en ce que**

a) dans une première étape la dispersion colorante est produite en mélangeant à une température de 30°C à 60°C au moins un colorant avec au moins un solvant et si nécessaire avec d'autres constituants en une dispersion et réduction subséquente du mélange par des appareils disperseurs et des instruments de broyage tels que des moulins, des homogénéisateurs Turrax ou d'autres mélangeurs puissants à une grandeur de particules de mains de 30 μm, ensuite
b) dans une seconde étape la substance tensioactive est préparée a une température de 40°C á 80°C par une dissolution aqueuse, ce après quoi
c) dans une troisième étape l'addition de la substance tensioactive produite dans la seconde étape à la dispersion colorante produite dans la première étape et tempérée a 30°C-60°C a lieu à une température de 30°C à 60°C,

cependant que l'on n'utilise pas de solvants organiques pour la fabrication du mélange colorant.

**2.** Procédé selon la revendication 1, **caractérise en ce**
**que** le mélange contient de plus des substances auxiliaires et/ou des additifs qui sont sélectionnés dans le groupe comprenant les rehausseurs de goût, les matières aromatiques ou les conservateurs.

**3.** Procédé selon la revendication 1 ou la revendication 2, **caractérise en ce que** le mélange contient de plus des supports qui sont sélectionnés dans le groupe comprenant les polysaccharides, les maltodextrines et les polyols.

**4.** Procédé selon l'une des revendications précédentes, **caractérise en ce que** la température de mélange lors du mélange de la dispersion colorante et de la substance tensioactive est de 30 à 50°C, de préférence de 40 à 50°C et la durée de mélange 15 a 90 minutes, de préférence de 30 à 60 minutes.

5. Procédé selon l'une des revendications précédentes, **caractérise en ce que** la dispersion colorante est tempérée, lors de l'addition de la seconde composante, de 30 à 50°, de préférence de 35 à 45°C et de manière particulièrement particulièrement préférée à 40°C.

6. Mélange colorant obtenu par le procédé selon l'une des revendications 1 à 5 comprenant

a) une dispersion colorante avec un solide qui est un caroténoïde tel que du carotène et des caroténoïdes d'origine identique au naturel ou d'origine naturelle, un bétadine, un riboflavine, un anthocyane, un produit de carmine, un curcuminoïde, un porphyrène et/ou un composé de chlorophylle, un composé de chlorophylline, un composé de cuivre/chlorophylle et/ou cuivre/chlorophylline avec une grandeur de particules moyenne de moins de 30 $\mu$m et avec un agent auxiliaire ou un additif qui est un sucre, un polysaccharide, un hydrocolloïde et de l'eau ou un antioxydant ou un conservateur et
b) une surface tensioactive qui est un émulateur autorisé dans les aliments ou les additifs alimentaires tels que les colorants, à savoir une lécithine, polysorbate 80, Lactem ou Citrem, la substance tensioactive étant contenue dans le mélange colorant dans une proportion inférieure à 20%, de préférence inférieure à 10% et de manière particulièrement préférée de 1% à 5% par rapport au mélange.

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 3206316 A
- US 6007856 A
- US 4522743 A
- US 5827539 A
- US 4726955 A
- WO 0070967 A
- US 4307117 A
- US 6132790 A